# FASCICULE DE BREVET EUROPEEN

(11) **EP 0 414 883 B1**
(45) Date de publication et mention de la délivrance du brevet: **15.06.1994**
(21) Numéro de dépôt: 90905550.1
(22) Date de dépôt: 20.03.1990
(51) Int. Cl.: A61M 5/50

(54) **SERINGUE HYPODERMIQUE A UTILISATION UNIQUE**
SPRITZE FÜR EINE SUBKUTANE INJEKTION OHNE WIEDERVERWENDUNGSMÖGLICHKEIT
SINGLE-USE HYPODERMIC SYRINGE

(30) Priorité: 20.03.1989 FR 8903592
(43) Date de publication de la demande: 06.03.1991
(73) Titulaire: HAMMAMI, Alain, F-75009 Paris (FR)
(72) Inventeur: HAMMAMI, Alain, F-75009 Paris (FR)
(74) Mandataire: Cabinet HERRBURGER
(86) Numéro de dépôt international: FR9000188
(87) Numéro de publication internationale: WO9011100

(56) Documents cités:
- EP-A- 0 291 109
- EP-A- 0 345 159
- CH-A- 255 256
- FR-A- 1 039 984
- FR-A- 2 412 320
- GB-A-11 509 80
- US-A- 4 687 467
- US-A- 4 690 154
- US-A-47 753 64

## Description

La présente invention concerne une seringue hypodermique à utilisation unique selon le préambule de la revendication 1.

Bien que cette seringue, très répandue, fabriquée et utilisée à des dizaines de millions d'exemplaires par an soit une seringue dite à jeter, rien dans la structure de cette seringue ne la rend inutilisable après une première utilisation.

Cette seringue connue est considérée comme une seringue à jeter plus qu'une seringue à usage unique car elle est réalisée principalement en matière synthétique, à bon marché et non comme les seringues anciennes non jetables dont on changeait seulement l'aiguille.

Or, avec le développement des maladies graves transmissibles par le sang, il devient nécessaire de prévoir une seringue qui, par sa construction, ne soit pas réutilisable.

Il existe déjà une seringue jetable du type de celle ci-dessus (US-A- 4 687 467). Cette seringue comporte un prolongement arrière tranchant, à l'aiguille, venant en saillie dans le cylindre. Lorsque le piston vient en fin de course, à l'éjection du liquide, son embout s'empale sur l'extrémité arrière tranchante de l'aiguiller. Grâce à la structure creuse du téton d'entraînement, les deux chambres du cylindre communiquent ce qui interdit toute réutilisation de la seringue.

Cette seringue peut également être réutilisée si le premier utilisateur (volontairement ou involontairement) ne vide pas complètement la seringue, c'est-à-dire aspire un volume de liquide supérieur à celui qu'il aura à injecter.

De plus, cette seringue nécessite une réalisation particulière de la partie arrière de l'aiguille, de l'embout et surtout une forme compliquée de l'extrémité du piston c'est-à-dire du téton d'entraînement et de son pion.

On connaît déjà une seringue hypodermique à utilisation unique, cette seringue comportant :
- un cylindre recevant un piston muni d'un embout en forme de corps cylindrique creux ayant une paroi périphérique assurant l'étanchéité par rapport au cylindre, une face antérieure fermée, une face postérieure munie d'un orifice pour fixer l'embout sur le piston,
- la face frontale fermée de l'embout ayant une paroi destinée à être percée par un dard porté par le piston sous l'action du mouvement de poussée du piston,
- le piston comporte un flasque contre lequel s'appuie l'embout sous l'effet de la poussée du piston,
- le piston comporte un téton d'entraînement avec un pion recevant l'embout et porte sur son pion le dard.

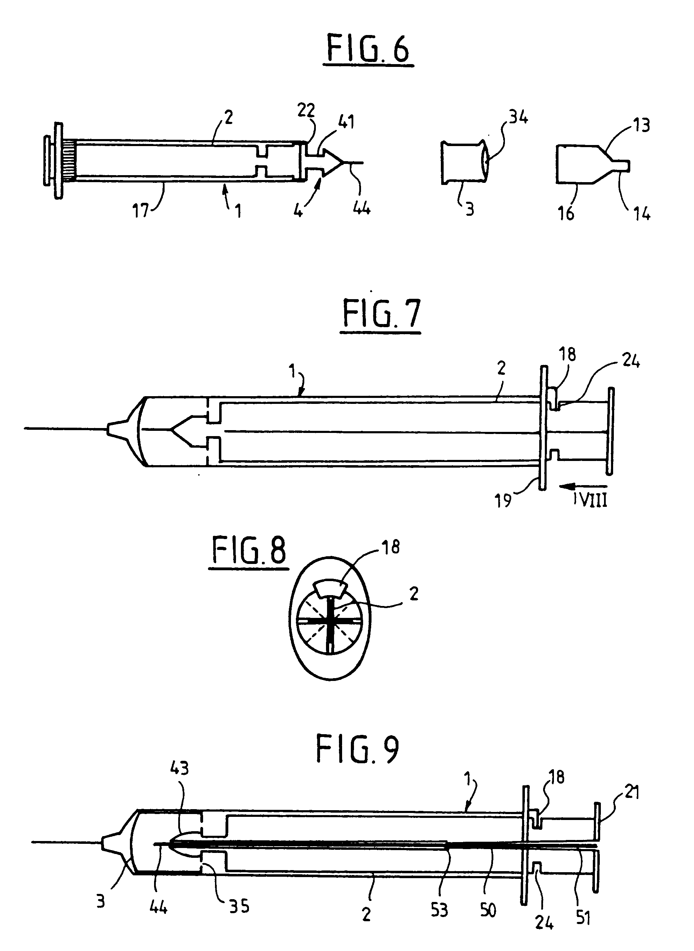

Une telle seringue est décrite au document EP - 0 345 159 (CLOTTEAU/POZZI) qui représente un état de la technique selon l'article 54(3). Toutefois, bien que cette seringue soit réellement à usage unique puisque la moindre pression exercée sur le piston pour expulser du liquide de la seringue crève la paroi par le dard (figures 7, 8) et rend la seringue inutilisable une seconde fois, cette seringue est une solution théorique qui ne fonctionne pas en pratique puisque sous l'effet de la pression exercée par le piston sur le liquide, ce liquide fuit à travers la crevure de la paroi et les canaux du téton et ceux du piston. Comme il n'y a pas de moyens d'étanchéité en aval de la crevure, cette seringue est inutilisable.

La présente invention a pour but de remédier à ces inconvénients des solutions connues et se propose de développer, à partir des seringues usuelles, une seringue qui soit réellement à usage unique, tout en permettant de bénéficier des conditions de fabrication particulièrement efficaces et économiques de seringues connues.

A cet effet l'invention concerne une seringue du type ci-dessus caractérisée par les caractéristiques de la pointe caractérisante de la 1ère revendication.

La seringue selon l'invention est intrinsèquement une seringue à usage unique car dès que l'utilisateur exerce une poussée sur la tige du piston, le dard crève la paroi de la face frontale de l'embout. Cette conséquence est inévitable quelles que soient les dispositions prises par l'utilisateur même mal intentionné qui souhaiterait pouvoir réutiliser la seringue ou utiliser une seringue jetée.

D'autres caractéristiques de l'invention font l'objet des revendications dépendantes.

L'invention sera mieux comprise à la lecture de la description faite en référence aux dessins dans lesquels :
- la figure 1 est une vue d'une seringue selon l'invention ;
- la figure 2 est une vue partielle à plus grande échelle de la figure 1 ;
- la figure 3 est une vue arrière de l'embout ;
- la figure 4 est une vue partielle de l'extrémité postérieure ouverte du cylindre ;
- la figure 5 est une vue suivant la flèche V de la figure 4 ;
- la figure 6 schématise le montage de la seringue selon l'invention ;
- la figure 7 est une vue d'une variante de réalisation de la seringue selon l'invention ;
- la figure 8 est une vue selon la flèche VIII de la figure 7 ;
- la figure 9 est une vue en coupe de la seringue selon une seconde variante de réalisation.

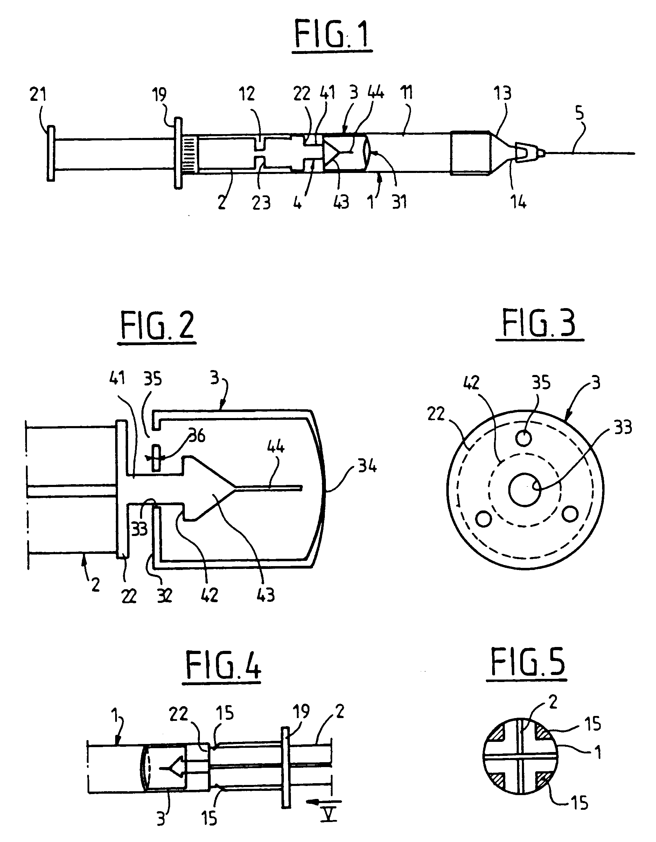

La figure 1 montre la seringue hypodermique qui présente un cylindre 1 dont une extrémité ouverte est bordée par une collerette 19 ; l'autre extrémité comporte un fond 13 conique se terminant par une tubulure ouverte 14, de petites dimensions destinée à recevoir l'aiguille 5.

Le cylindre 1 reçoit un piston 2 mobile axialement ; le piston porte une face plane 21, sur laquelle agit l'utilisateur de la seringue, à son extrémité externe au cylindre ; son extrémité située dans le cylindre 1 présente un téton d'entraînement 4 par lequel le piston porte un embout creux 3.

L'embout 3 réalisé en une matière déformable, se plaque par sa périphérie externe contre la paroi interne du cylindre 1 et le divise de manière étanche en deux chambres 11, 12.

Selon la figure 2, le téton d'entraînement 4 du piston 2 présente successivement :
- un flasque 22 transversal,
- une tige 41 s'étendant depuis le flasque 22 dans l'axe longitudinal de la seringue, vers l'extrémité avant de la seringue,
- à l'extrémité de la tige 41, un pion 43, de forme générale conique ou cylindro-conique, présentant du côté arrière une face transversale 42, et du côté avant un dard 44.

L'embout 3 en matière déformable, par exemple une matière caoutchouteuse, se présente globalement sous la forme d'un cylindre creux, dont la face antérieure 31 est fermée tandis que la face postérieure 32 présente une ouverture 33 axiale, d'un diamètre approximativement égal à celui de la tige 41 du téton d'entraînement.

La face antérieure fermée 31 de l'embout 3 est d'épaisseur variable ; sa partie centrale 34 est de faible épaisseur afin d'être facilement déchirée ou percée. Cette partie 34 fragile est obtenue, soit d'une seule pièce en moulant une paroi mince, soit en réalisant un embout ayant une ouverture dans la partie centrale 34 de la face avant, et en rapportant sur cette ouverture une paroi de faible épaisseur, fragile, du type pastille, par exemple soudée aux ultrasons ou par tout autre moyen connu.

La face 32 comporte des ouvertures 35, ici au nombre de trois.

Comme l'illustre la figure 3, ces ouvertures 35 sont réparties circonférentiellement et sont centrées sur une circonférence moyenne entre le disque couvert par le flasque 22 et celui couvert par la face transversale 42 du pion 43.

L'épaisseur de la paroi arrière 36 de l'embout 3 est sensiblement inférieure à la longueur de la tige 41 afin d'autoriser un déplacement axial de l'embout le long de la tige 41 entre le flasque 22 et la face 42. Ainsi, pour une portion déterminée de l'embout dans la seringue, le piston peut se déplacer librement entre deux extrémités de la tige 41.

La seringue est fabriquée et commercialisée avec le piston 2 enfoncé dans le cylindre afin que l'embout 3 ait sa face 31 proche du fond 13 dudit cylindre.

Lors de la première utilisation, on tire le piston 2 dans la direction tendant à le sortir du cylindre 1 pour aspirer le liquide à injecter. Au cours de cette phase, l'embout 3 est en appui par sa face 32 contre la face 42 du téton d'entraînement 4.

Lorsque l'on repousse le piston en direction du fond 13 du cylindre, il se produit tout d'abord un déplacement du piston 2 par rapport à l'embout 3 jusqu'à ce que le piston 2 appuie par son flasque 22 sur la face 32 de l'embout.

Au cours de ce déplacement relatif du piston et du téton d'entraînement par rapport à l'embout 3, le dard 44 situé initialement à faible distance de la face 31, vient perforer la portion fragile 34 de la face 31 de l'embout 3. Le perçage est de plus assuré par la déformation de la face 31 du fait de la résistance que lui oppose le liquide lorsque l'on pousse le piston.

Lors du déplacement du piston 2 vers le fond du cylindre, le flasque 22 est au contact de la face 32 de l'embout et obture ainsi les ouvertures 35, la seringue fonctionne ainsi comme une seringue de type connu et expulse le liquide contenu dans la chambre 11 par l'aiguille 5.

Jusqu'ici, le fonctionnement de la seringue a été au moins, du point de vue de l'utilisateur, le même que celui d'une seringue de type connu. Toutefois, sans que l'utilisateur ne s'en aperçoive et n'ait à le provoquer et, de plus, de manière tout à fait intéressante sans qu'il puisse l'éviter, l'embout 3 a été percé par le dard 44.

Afin d'assurer l'étanchéité entre le flasque 22 et la face 32, il est nécessaire que les ouvertures 35 soient fermées de manière sûre. Le cas échéant, il est possible de prévoir une collerette dépassant de la face 32 et tournée vers le flasque 22 ; la collerette a un diamètre suffisant pour cacher les ouvertures 35.

Comme la seringue ne peut pas être de nouveau remplie de liquide puisque l'embout 3 met les deux chambres 11, 12 en communication, elle ne peut donc pas être réutilisée.

Afin d'empêcher l'utilisateur de retirer le piston 2 pour remplir le cylindre 1 par son extrémité ouverte, des moyens de verrouillage sont prévus dans le cylindre du côté de son extrémité ouverte.

Comme illustré aux figures 4 et 5, ces moyens de verrouillage peuvent se présenter sous la forme de languettes 15 obtenues à partir de crevés de la paroi du cylindre 1 ; ces languettes s'écartent de la paroi vers l'axe du cylindre en se rapprochant du fond dudit cylindre.

Les languettes 15 sont élastiques, ce qui permet de les écarter pour entrer le piston 2 dans le cylindre puis, une fois le flasque 22 positionné entre lesdites languettes et le fond du cylindre, tout mouvement tendant à sortir le piston du cylindre sera arrêté par l'arrivée en butée dudit flasque 22 contre les languettes.

Comme le montre la figure 5, le piston 2 présentant dans sa partie courante une section transversale en forme de croix, les languettes pourront être au nombre de quatre, chacune étant située dans un quadrant laissé libre par le piston.

Les moyens d'arrêt pourraient, bien entendu, être de toute autre conception. A titre d'exemple, on peut créer un bourrelet à l'intérieur du cylindre 1. A cet effet, la collerette 19 du cylindre 1 est rapportée sur l'extrémité de celui-ci et présente un pourtour interne dépassant à l'intérieur du cylindre qui soit élastique afin de permettre l'enfoncement du piston 2 lors de sa mise en place.

La seringue ne peut en aucune façon être remplie de nouveau lorsqu'elle a déjà été utilisée une première fois puisque l'embout n'assure plus l'étanchéité du piston dans le cylindre.

Afin d'éviter de manière encore plus sûre la réutilisation de la seringue, on prévoit, en plus du perçage de l'embout interdisant l'aspiration du liquide, un moyen de destruction partielle de la seringue.

A cet effet, le piston 2 comporte, à peu de distance du flasque 22 d'extrémité, une rainure circonférentielle 23 qui fragilise le piston et permet à l'utilisateur de le casser après la perfusion. Une fois la portion du piston 2 sortant à l'extérieur du cylindre cassé, il devient impossible de déplacer la portion restante portant l'embout 3 dans le sens nécessaire pour aspirer le liquide.

Un mode de fabrication de la seringue sera décrit ci-après. Il est essentiel de faire le montage avec beaucoup de soin afin de ne pas percer la paroi fragile 34.

Dans un premier mode de réalisation, illustré à la figure 6, le cylindre 1 est prévu en deux parties, l'une 16, de faible longueur, à l'avant de la seringue, comportant le fond 13 et le nez 14, et l'autre 17, la plus grande, comportant le tube cylindrique, l'extrémité arrière ouverte et la collerette 19 qui l'entoure.

Le piston 2 est introduit dans la portion 17 jusqu'à ce que sa face transversale 22 affleure la face d'extrémité avant de ladite portion 17 de cylindre 1. L'embout 3 est alors monté sur le téton d'entraînement 4, la longueur du dard 44 est prévue de manière qu'au repos, il soit à une distance suffisante de la paroi fragile 34. Afin d'être certain de ne pas enfoncer l'embout 3 jusqu'à ce qu'il soit contre le flasque 22, ce qui risquerait d'amener le dard 44 à percer la paroi fragile 34, on peut amener sur la tige un gabarit arrêtant le mouvement.

On peut, en outre, prévoir un embout de longueur sensiblement supérieure à la longueur du dard 44, de sorte que la paroi fragile 34 reste éloignée du dard. En variante, on peut prévoir un embout dont la membrane présente à l'avant un orifice et, après montage sur le téton d'entraînement, rapporter une paroi fragile 44 dans cet orifice.

Une fois l'embout 3 ainsi mis en place sur le piston 2, on enlève le gabarit, si on l'a utilisé, puis on coiffe ledit embout avec la portion 16 de cylindre que l'on fixe à la portion 17 par tout moyen connu tel que collage, soudage à ultra-sons ...

On voit que la portion 16 a une longueur légèrement supérieure à l'embout 3 afin que celui-ci soit proche du fond 13 mais le le touche pas pour ne pas risquer le perçage de la paroi 34 rendant alors la seringue inutilisable.

Afin de ne pas risquer une manipulation intempestive du piston, par exemple en direction du fond 13 de la seringue, ce qui crèverait la paroi 34 avant l'utilisation de ladite seringue, on prévoit des moyens d'arrêt 18, 24 tels qu'il soit nécessaire de suivre une procédure précise lors de l'utilisation de la seringue pour déplacer le piston 2.

Tels que visibles aux figures 7 et 8, les moyens d'arrêt peuvent être formés d'un ergot 18 solidaire du cylindre 1 positionné dans une encoche 24 du piston 2. Lorsque le piston 2 est positionné dans la position représentée en traits pleins à la figure 8, il est impossible de le déplacer axialement dans le cylindre. Pour utiliser la seringue, il est nécessaire de faire tourner le piston 2 sur lui-même afin de l'amener dans la position représentée en pointillés à la figure 8 pour laquelle l'ergot 18 ne coopère plus avec l'encoche 24.

De préférence, dans la forme de réalisation représentée, le piston ayant la forme d'un croisillon, chaque aile comporte une encoche 24 et l'ergot a une forme triangulaire d'angle au sommet légèrement inférieur à 90°. Ainsi, le déverrouillage du piston n'est réalisé qu'après une rotation importante, 90° au maximum, ce qui évite le déverrouillage par inadvertance.

La seringue selon l'invention est fabriquée avec les matériaux usuels déjà approuvés par les instances médicales sans nécessiter de nouveaux choix. Notamment, le dard 44 ou l'aiguille peuvent être réalisés en matière synthétique, identique à celle du corps du piston. On est ainsi certain que ces matériaux soient adaptés à l'utilisation médicale que l'on veut en faire.

## Revendications

1. Seringue hypodermique à utilisation unique comportant :
- un cylindre (1) recevant un piston (2) muni d'un embout (3) en forme de corps cylindrique creux ayant une paroi périphérique assurant l'étanchéité par rapport au cylindre (1), une face antérieure (31) fermée, une face postérieure (32) munie d'un orifice (33) pour fixer l'embout (3) sur le pion (43) d'un téton d'entraînement (4) du piston (2), la face frontale fermée (31) de l'embout (3) ayant une paroi (34), destinée à être percée par un dard (44) porté par le piston (2), sous l'action du mouvement de poussée du piston (2),
- le piston (2) comporte un téton d'entraînement (4) avec un pion (43) recevant l'embout (3), seringue caractérisée en ce que :
- le téton d'entraînement (4) porte sur son pion (43) le dard (44),
- la face postérieure (32) de l'embout comporte en plus de l'orifice (33), au moins une autre ouverture (35) située hors du champ de contact du téton d'entraînement (4) mais à l'intérieur du disque couvert par un flasque (22),
- l'ouverture (35), mettant l'intérieur de l'embout (3) en communication avec une chambre arrière (12) du cylindre (1) lorsqu'on exerce une traction sur le piston.

2. Seringue selon la revendication 1, caractérisée en ce que le téton d'entraînement (4) comporte une tige (41) reliant le pion (43) à un flasque d'extrémité (22) du piston (2).

3. Seringue selon la revendication 2, caractérisée en ce que la tige (41) du téton d'entraînement (4) est substantiellement plus longue que l'épaisseur de l'embout (3) afin que l'embout (3) se déplace entre une première position pour laquelle sa face postérieure (32) vient contre la face transversale (42) du pion (43) du téton d'entraînement (4) au moment de la traction de remplissage exercée sur le piston et une autre position pour laquelle la face postérieure (32) est appliquée contre le flasque d'extrémité (22) lors du refoulement du liquide de la seringue au moment de l'injection.

4. Seringue selon la revendication 3, caractérisée en ce que le dard (44) perfore la paroi fragile (34) au moment de la poussée de refoulement exercée par l'intermédiaire du piston sur le liquide de la seringue, l'étanchéité de l'embout étant assurée par l'obturation des ouvertures (35) par le flasque d'extrémité (22) alors qu'en position de retrait lorsqu'une traction est exercée sur le piston, le dard (44) est à une certaine distance de la paroi fragile (34).

5. Seringue selon l'une quelconque des revendications 1 à 4, caractérisée en ce qu'elle comporte des moyens d'arrêt (15) qui interdisent le retrait du piston (2) ) l'extérieur du cylindre (1).

6. Seringue selon l'une quelconque des revendications 1 à 5, caractérisée en ce que le piston (12) est fragilisé par une rainure circonférentielle (23) permettant de casser le piston.

7. Seringue selon l'une quelconque des revendications 1 à 6, caractérisée en ce que le cylindre (1) est réalisé en deux parties (16, 17) assemblées l'une à l'autre.

8. Seringue selon l'une quelconque des revendications 1 à 7, caractérisée par des moyens d'arrêt (18, 24) qui bloquent tout déplacement du piston (2) tant qu'une procédure de mise en oeuvre n'a pas été suivie.

## Claims

1. A single-use hypodermic syringe comprising :
- a cylinder (1) receiving a plunger (2) provided with a cap (3) in the form of a hollow cylindrical body having a peripheral wall assuring water-tightness with regard to the cylinder (1), a closed front face (31), a rear face (32) provided with an opening (33) for fixing the cap (3) on the head (43) of a retaining stud (4) of the plunger (2), the closed front face (31) of the cap (3) having a wall (34) intended to be pierced by a pin (44) carried by the plunger (2) under the action of the pressing movement of the plunger (2),
- the plunger (2) comprises a retaining stud (4) having a head (43) receiving the cap (3),
characterised in that :
- the retaining stud (4) carries the pin (44) on its head (43),
- the rear face (32) of the cap comprises in addition to the opening (33) at least one other opening (35) situated outside the field of contact of the retaining stud (4) but on the inside of the disc covered by a disc (22),
- the opening (35), placing the inside of the cap (3) in communication with a rear chamber (12) of the cylinder (1) when traction is exerted on the plunger.

2. A syringe according to claim 1, characterised in that the retaining stud (4) comprises a shaft (41) connecting the head (43) to an end disc (22) of the plunger (2).

3. A syringe according to claim 2, characterised in that the shaft (41) of the retaining stud (4) is substantially longer than the thickness of the cap (3) so that the cap (3) moves between a first position for which position for which position its roar face (32) comes against the transverse face (42) of the head (43) of the retaining stud (4) at the moment of filling traction exerted on the plunger and another position for which position the rear face (32) is brought against the end disc (22) when the liquid is expelled out of the syringe at the moment of injection.

4. A syringe according to claim 3, characterised in that the pin (44) perforates the fragile wall (34) at the moment of expulsion pressure exerted by means of the plunger on the liquid in the syringe, the water-tightness of the cap being assured by the sealing of the openings (35) by the end disc (22) although in the position of retraction when a a traction is exerted on the plunger the pin (44) is at a safe distance from the fragile wall (34).

5. A syringe according to any one of the claims 1 to 4, characterised in that it comprises the means of stopping (15) which provent the retraction of the plunger (2) to the exterior of the cylinder (1).

6. A syringe according to any one of the claims 1 to 5, characterised in that the plunger (2) is rendered fragile by a circunferential groove (23) allowing the plunger to be broken.

7. A syringe according to any one of the claims 1 to 6, characterised in that the cylinder (1) is produced in two parts (16, 17) assembled one onto another.

8. A syringe according to any one of claims 1 to 7, characterised by the means of stopping (18, 24) which block any movement of the plunger (2) as long as a process of implementation has not been followed.

## Patentansprüche

1. Einmalspritze für subkutane Injektion, bestehend aus:
- einem Zylinder (1), der einen Kolben (2) mit zylindrischem hohlem Ansatz (3) trägt, dessen Aussenwand die Abdichtung gegenüber dem Zylinder (1) gewährleistet, mit einer geschlossenen Vorderwand (31), mit einer durch eine Öffnung (33) versehenen Hinterwand (32), um den Ansatz (3) auf die Scheibe (43) des beweglichen Zapfens (4) des Kolbens (2) zu tragen, wobei die geschlossene Vorderseite (31) des Ansatzes (3) mit einer Wand (34) versehen ist, die bei der Schubbewegung des Kolbens (2) durch eine vom Kolben (2) getragene Nadel (44) durchbohrt wird,
- der Kolben (2) trägt einen beweglichen Zapfen (4) mit einer Scheibe (43), der den Ansatz (3) trägt,
wobei die Injektionsspritze dadurch gekennzeichnet ist, daß
- die Nadel (44) auf der Scheibe (43) des beweglichen Zapfens (4) angebracht ist,
- zusätzlich zur Öffnung (33) die Hinterwand (32) des Ansatzes mit mindestens einer anderen Öffnung (35) außerhalb der Kontaktfläche des beweglichen Zapfens (4) versehen ist, die sich jedoch innerhalb der Scheibenfläche eines Flansches (22) befindet,
- durch die Öffnung (35) der Innenraum des Ansatzes (3) mit einer Hinterkammer (12) des Zylinders (1) in Verbindung gebracht wird, wenn der Kolben herausgezogen wird.

2. Injektionsspritze nach Anspruch 1,
dadurch gekennzeichnet,
daß der bewegliche Zapfen (4) eine Stange (41) trägt, die die Scheibe (43) mit einem Befestigungsflansch (22) des Kolbenendes (2) verbindet.

3. Injektionsspritze nach Anspruch 2,
dadurch gekennzeichnet,
daß die Stange (41) des beweglichen Zapfens (4) wesentlich länger im Verhältnis zur Dicke des Ansatzes (3) ist, damit der Ansatz zwischen einer ersten Endlage bewegen kann, wo sein hinteres Teil (32) gegen die Seitenfläche (42) der Scheibe (43) des beweglichen Zapfens (4) angedrückt wird, wenn der Kolben für die Abfüllung gezogen wird und einer anderen Endlage, wo der hintere Teil (32) gegen den Befestigungsflansch (22) angedrückt wird, wenn die Flüssigkeit während der Injektion angestaut wird.

4. Injektionsspritze nach Anspruch 3,
dadurch gekennzeichnet,
daß die Nadel (44) die zerbrechliche Wand (34) unter dem Staudruck durchbohrt, wenn der Kolben gegen die Flüssigkeit innerhalb der Spritze gedrückt wird, wobei die Dichtheit des Ansatzes durch die Schliessung der Öffnungen (35) durch den Befestigungsflansch (22) gewährleistet wird, während sich die Nadel (44) in einer gewissen Entfernung zur zerbrechlichen Wand (34) befindet, wenn der Kolben herausgezogen wird.

5. Injektionsspritze nach einem beliebigen der Ansprüche 1 bis 4,
dadurch gekennzeichnet,
daß sie mit Arretierungsmitteln (15) versehen wird, die ein Herausspringen des Kolbens (2) außerhalb des Zylinders (1) verhindern.

6. Injektionsspritze nach einem beliebigen der Ansprüche 1 bis 5,
dadurch gekennzeichnet,
daß der Kolben (12) durch eine Kreisnut (23) auf seinen gesamten Umfang leicht zerbrechlich gemacht wurde.

7. Injektionsspritze nach einem beliebigen der Ansprüche 1 bis 6,
dadurch gekennzeichnet,
daß der Zylinder (1) aus zwei zusammengefügten Teilen (16,17) besteht.

8. Injektionsspritze nach einem beliebigen der Ansprüche 1 bis 7,
dadurch gekennzeichnet,
daß Arretierungsmittel (18,24) vorhanden sind, die jede Bewegung des Kolbens (2) solange verhindern, bis eine Anwendungshandlung ausgeführt wird.
